# EUROPEAN PATENT APPLICATION

(11) **EP 0 640 620 A2**
(43) Date of publication of application: **01.03.1995**
(21) Application number: 94304793.6
(22) Date of filing: 30.06.1994
(51) Int. Cl.: C07K 14/705, C07K 16/28, G01N 33/50, G01N 33/566

(54) **Methods and reagents for inhibiting cholesterol absorption in humans mediated by pancreatic esterase receptor protein**

(30) Priority: 01.07.1993 US 86517
(71) Applicant: Lange, Louis G., III, Portola Valley, California 94028 (US); Bosner, Matthew S., Chesterfield, Missouri 63017 (US)
(72) Inventor: Lange, Louis G., III, Portola Valley, California 94028 (US); Bosner, Matthew S., Chesterfield, Missouri 63017 (US)
(74) Representative: Eyles, Christopher Thomas

(57) **Abstract**

This invention relates to the human enzyme, pancreatic cholesterol esterase (PCE), and a receptor (PCE^{R}) for this protein expressed by cells of the intestinal lining. Specifically, the invention relates to the use of inhibitors of binding between PCE and PCE^{R} to reduce intestinal uptake of dietary cholesterol in humans. Antibodies, including antisera, and monoclonal and chimeric antibodies, and cell lines producing such antibodies, raised against the PCE^{R} protein or fragments or epitopes thereof, are also provided. There is also described treatment of individuals with therapeutic drugs for the prevention of alleviation of disease states in a human related to cholesterol metabolism.

## Description

### BACKGROUND OF THE INVENTION

### 1. Field of the Invention

This invention relates to dietary cholesterol absorption in humans and methods for inhibiting this absorption. In particular, the invention relates to a human enzyme, pancreatic cholesterol esterase (PCE), known to be important in facilitating dietary cholesterol absorption, and a receptor (PCE^{R}) for this protein expressed by cells of the intestinal lining. Specifically, the invention relates to the use of inhibitors of binding of the PCE receptor with PCE to reduce intestinal uptake of dietary cholesterol in humans. Antibodies, including antisera, and monoclonal and chimeric antibodies, and cell lines producing such antibodies, raised against the PCE^{R} protein or fragments or epitopes thereof, are also provided. The invention also relates to treatment of individuals with therapeutic drugs for the prevention or alleviation of disease states in a human related to cholesterol metabolism.

Cholesterol metabolism is of critical interest to those involved in protecting human health. Atherosclerosis is the leading cause of death in the United States and reduction of serum cholesterol levels has recently been embraced as a national health priority. See NIH Consensus Panel Report, J.A.M.A. 253: 2094 (1985). NIH recommendations include measurement of serum cholesterol in all adults, with efforts to reduce cholesterol in those individuals with levels above 200 mg%. In this regard front line therapy is a reduction in the amount of cholesterol and triglycerides ingested, followed by the use of agents that interfere with absorption of ingested lipids. See Consensus Full Report, Arch. Inst. Med. 148: 36 (1988).

Since about 90% of dietary cholesterol is comprised of free cholesterol, it is not obvious that pancreatic cholesterol esterase should be important in dietary cholesterol absorption in humans. In fact, it had been thought prior to this time that cholesterol esterase was not important for cholesterol absorbsion [*see. for example*. Huang and Hiu, J. Lipid Res. 31: 2029 (1991)]. Unexpectedly, pancreatic cholesterol esterase is now known to play a pivotal role in the absorption of cholesterol and fatty acids (U.S. Patent No. 5,017,565, issued May 21, 1991). Pancreatic cholesterol esterase is known to interact with and be bound to cells lining the intestinal brush border (Bosner *et al.*, 1988, Proc. Natl. Acad. Sci. USA 85: 7438-7442) *via* a receptor-like interaction with brush border membrane-associated heparin. Human (Nilsson *et al*., 1990, Eur. J. Biochem. 192: 323-326; Kumar *et al.*, 1992, Biochemistry 31: 6077-6081), rat (Kissel *et al.*, 1989, Biochim. Biophys. Acta 1006: 227-236) and bovine (Kyger *et al.*, 1989, Biochem. Biophys. Res. Comm. 164: 1302-1309) PCE genes have recently been isolated and characterized (*see* co-pending U.S. Patent Application Serial No. 07/730,204, filed July 15, 1991), and comprise a heparin binding site. These results suggest that the association between PCE and the intestinal brush border is mediated by binding to a specific, heparin-containing receptor for PCE. Disruption of PCE-receptor binding should lead to the inhibition or abrogation of PCE-facilitated dietary cholesterol processing and uptake, thereby helping to alleviate diseases having an elevated serum cholesterol etiology.

### 2. Background of the Related Art

Borja *et al.*, 1964, Proc. J. Exp. Biol. and Med. 116: 496 teach that cholesterol esterase is secreted by the pancreas, and that its catalysis of cholesterol ester hydrolysis to produce free cholesterol and free fatty acids is essential for the absorption of cholesterol derived from cholesterol esters.

Norum *et al*., 1983, Physiol. Rev. 63: 1343-1419 review the biochemistry of cholesterol absorbsion and metabolism, including the role of pancreatic cholesterol esterase.

Bosner *et al.*, *ibid.* teach that cholesterol esterase performs its function while anchored to the intestinal membrane via a receptor-like interaction with brush border membrane-associated heparin.

Kyger *et al., ibid.*, teach the nucleic acid sequence of a cDNA clone of bovine pancreatic cholesterol esterase.

Kissel *et al.*, *ibid.*, teach the nucleic acid sequence of a cDNA clone of mRNA encoding pancreatic cholesterol esterase of the rat.

Nilsson *et al.*, *ibid.*, teach the nucleic acid sequence of a partial cDNA clone of human pancreatic cholesterol esterase.

Taylor *et al.*, 1991, Genomics 10: 425-431 disclose the localization of the human cholesterol esterase gene to the terminal region of the long arm of chromosome 9.

Kumar *et al.*, *ibid.*, teach the structure and nucleic acid sequence of the human pancreatic cholesterol esterase gene.

### BRIEF SUMMARY OF THE INVENTION

This invention relates to reagents for the inhibition of dietary cholesterol uptake in humans. The invention provides the human pancreatic cholesterol esterase receptor protein as a homogeneous composition of matter. The invention also provides methods utilizing this receptor protein for isolating and characterizing compounds capable of inhibiting or modulating binding between the receptor and pancreatic cholesterol esterase. Antisera and antibodies, including monoclonal and chimeric antibodies, as well as cell lines producing such antibodies, raised against the PCE^{R} protein or fragments or epitopes thereof, are also provided. Therapeutic methods using pharmaceutical compositions of appropriate inhibitory and modulator compounds isolated and characterized according to the methods herein are also described.

### DETAILED DESCRIPTION OF THE PREFERRED EMBODIMENTS

This invention provides a homogenous composition of matter comprising a mammalian pancreatic cholesterol esterase receptor (PCE^{R}), preferably human PCE^{R}. This protein can be isolated using the methods described herein from any mammalian cell line or tissue that expresses the receptor, and is preferably isolated from the human colon carcinoma cell line CaCo-2 (American Type Culture Collection, ATCC, accession No. 4).

The PCE^{R} provided by the invention is characterized by having an apparent molecular weight of about 150K to about 160K, as determined for the native protein by sodium dodecyl sulfate/ polyacrylamide gel electrophoresis (SDS/PAGE). The receptor protein is a glycoprotein, as evidenced by the fact that treatment with glycosaminoglycosidases reduced the apparent molecular weight of the native protein to about 45-50K. Specifically, heparinase treatment liberates ³⁵S from the native molecule, indicating that heparin or a heparin derivative is covalently linked to the receptor *in vivo*.

PCE^{R} protein is isolated from plasma membrane fragments from CaCo-2 cells as described in the Examples that follow. The receptor protein was labeled with either Na[³⁵S] or [³H]-lysine or serine, or a combination of such ³⁵S and ³H labels, and membranes isolated using conventional biochemical separation techniques, including mechanical disruption of the cells and centrifugal separation of plasma membrane fractions. Such isolated membranes were then solubilized and subjected successively to DEAE chromatography and Sepharose C1-2B affinity chromatography, with radioactively-labeled fractions isolated and pooled before each successive step. The final recovered fraction was found to bind specifically to a cholesterol esterase affinity chromatography column, and was eluted in a solution of 4.2M NaCl/ 30mM sodium taurocholate.

Proteins provided by the present invention may be labeled to enable their detection. Labels used include but are not limited to radioactive labels (e.g., ³⁵S-labeled HSO₃⁻, ³H-labeled amino acids), fluorescent labels [e.g., fluorescein isothiocyanate (FITC)] and antigenic labels (e.g., biotin). Such labels are detected using techniques appropriate for detecting each label, including but not limited to autoradiography, fluorescence or by well-known immunologically-based techniques.

The invention also provides methods and reagents for isolating and characterizing agents capable of inhibiting or modulating the binding of PCE to PCE^{R}. In one embodiment of the invention, a complex mixture of small, bioactive molecules, such as a fungal broth or a peptide library, is contacted with the isolated PCE^{R} protein or with cells expressing the PCE receptor. Preferred cells are CaCo-2 cells. Binding assays between PCE^{R} molecules so contacted with a small molecule mixture and PCE are then performed to detect inhibition or modulation of PCE-PCE^{R} binding. PCE is provided by the invention either after biochemical isolation from naturally-occurring sources of such proteins or as the result of production by recombinant genetic means well known in the art. Optionally such proteins can be made as fusion proteins. The isolated PCE or PCE^{R} protein molecules can be advantageously provided bound to a solid support such as the inner surface of a microtitre plate or an agarose, acrylamide or sephadex bead. PCE can be optionally detectably labeled either metabolically with isotopes of hydrogen, carbon, phosphorus or sulfur, or by *in vitro* labeling with a radioactive label, an antigenic label, a hapten label, or a fluorescent label.

The small molecule mixture is incubated with isolated PCE^{R} protein or cells expressing PCE^{R} for a time and a temperature, for example 37°C, sufficient to enable specific binding of small molecules in the mixture to the PCE^{R} protein. The small molecule mixture is then removed and the PCE^{R}, having bound at least one species of small molecule from the mixture, is contacted with PCE and incubated for a time and a temperature, for example 37°C, sufficient to enable specific binding of the PCE ligand to the PCE receptor. The effect of the bound small molecules on ligand binding to PCE^{R} is then assayed, by comparing the amount of PCE protein bound by the PCE^{R} receptor in the presence and absence of incubation with the small molecule mixture. Bound small molecules whose binding results in an effect, either a diminution or inhibition of binding of PCE by PCE^{R}, are then eluted from the receptor molecules and can be further fractionated and characterized using conventional biochemical methods, such as high pressure liquid chromotagraphy. Small molecules so isolated are then assayed and characterized for their ability to inhibit or modulate PCE^{R} binding of PCE.

The invention also provides a method of screening a compound as an inhibitor of pancreatic cholesterol esterase receptor expression *in vivo*. In this embodiment, cells that express PCE^{R}, preferably CaCo-2 cells, are incubated with the putative inhibitor compound for a time sufficient to inhibit expression of the pancreatic cholesterol esterase receptor; for CaCo-2 cells, this effective time is from about 6h to about 24h. The cells are then incubated with isolated PCE protein molecules and PCE-PCE^{R} binding assayed as described above. For analyzing mixtures of small molecules, PCE inhibition binding assays are performed repetitively on such mixtures that are successively fractionated to greater homogeneity.

Also described are methods for administering the PCE^{R} binding inhibiting or modulating agents isolated and characterized using the methods of the invention to an animal, most preferably a human being. Applications of such methods include treatment for alleviating or preventing an elevated serum cholesterol-related disease in an animal. For this purpose, the inhibiting or modulating agents isolated and characterized as described herein are administered in a therapeutically active amount in a pharmaceutically effective carrier.

The invention also provides antibodies that are immunologically reactive to a mammalian, preferably human, PCE^{R} The antibodies provided by the invention can be raised in animals by inoculation with cells that express a mammalian PCE^{R} or epitopes of a mammalian PCE^{R} using methods well known in the art. Animals that can be used for such inoculations include individuals from species comprising cows, sheep, pigs, mice, rats, rabbits, hamsters, goats and primates. Preferred animals for inoculation are rodents (including mice, rats, hamsters) and rabbits. The most preferred animal is the mouse.

Cells that can be used for such inoculations, or for any of the other means used in the invention, include any cell line which naturally expresses a mammalian PCE^{R}, or any cell or cell line that expresses a mammalian PCE^{R} or any epitope therein as a result of molecular or genetic engineering, or that has been treated to increase the expression of a mammalian PCE^{R} by physical, biochemical or genetic means. Preferred cells are human cells, most preferably human CaCo-2 cells.

The present invention provides monoclonal antibodies that are immunologically reactive with an epitope that is a mammalian PCE^{R} present on the surface of mammalian cells, preferably human cells. These antibodies are made using methods and techniques well known to those of skill in the art. Monoclonal antibodies provided by the present invention are produced by hybridoma cell lines, that are also provided by the invention and that are made by methods well known in the art. Hybridoma cell lines are made by fusing individual cells of a myeloma cell line with spleen cells derived from animals immunized with cells expressing a mammalian PCE^{R}, including human cells, as described above. The myeloma cell lines used in the invention include lines derived from myelomas of mice, rats, hamsters, primates and humans. Preferred myeloma cell lines are from the mouse, and the most preferred mouse myeloma cell line is P3X63-Ag8.653. The animals from whom spleens are obtained after immunization are rats, mice and hamsters, preferably mice, most preferably Balb/c mice. Spleen cells and myeloma cells are fused using a number of methods well known in the art, including but not limited to incubation with inactivated Sendai virus and incubation in the presence of polyethylene glycol (PEG). The most preferred method for cell fusion is incubation in the presence of a solution of 45% (w/v) PEG-1450. Monoclonal antibodies produced by hybridoma cell lines can be harvested from cell culture supernatant fluids from *in vitro* cell growth; alternatively, hybridoma cells can be injected subcutaneously and/or into the peritoneal cavity of an animal, most preferably a mouse, and the monoclonal antibodies obtained from blood and/or ascites fluid.

Monoclonal antibodies provided by the present invention can also be produced by recombinant genetic methods well known to those of skill in the art, and the present invention encompasses antibodies made by such methods that are immunologically reactive with an epitope of a mammalian PCE^{R}.

The present invention encompasses fragments of the antibody that are immunologically reactive with an epitope of a mammalian PCE^{R}. Such fragments can be produced by any number of methods, including but not limited to proteolytic cleavage, chemical synthesis or preparation of such fragments by means of genetic engineering technology. The present invention also encompasses single-chain antibodies that are immunologically reactive with an epitope of a mammalian PCE^{R} made by methods known to those of skill in the art.

The present invention also encompasses an epitope of a mammalian PCE^{R} that is comprised of sequences and/or a conformation of sequences present in the mammalian, preferably human, PCE^{R} molecule. This epitope may be naturally occurring, or may be the result of proteolytic cleavage of the mammalian PCE^{R} molecule and isolation of an epitope-containing peptide or may be obtained by synthesis of an epitope-containing peptide using chemical methods well known to those skilled in the art. The present invention also encompasses epitope peptides produced as a result of genetic engineering technology and synthesized by genetically engineered prokaryotic or eukaryotic cells.

The invention also includes chimeric antibodies, comprised of immunologically reactive light chain and heavy chain peptides to an epitope that is a mammalian, preferably human, PCE^{R}. The chimeric antibodies embodied in the present invention include those that are derived from naturally occurring antibodies as well as chimeric antibodies made by means of genetic engineering technology well known to those of skill in the art.

The following Examples are shown by way of illustration and not by way of limitation.

### EXAMPLE 1

### Isolation and Characterization of the Human Pancreatic Cholesterol Esterase Receptor

The human pancreatic cholesterol esterase receptor protein was isolated from cell membranes from human CaCo-2 cells, a human colon carcinoma cell line (ATCC, Accession Number 4). Cells were grown in Eagle's MEM media (GIBCO, Long Island, NY) supplemented with 20% fetal calf serum (GIBCO) and 1% sodium pyruvate (GIBCO). Prior to isolation of the receptor proteins, cells were grown in sulfate-depleted media for 40 hr, and then 50 µCi/mL Na[³⁵S] and 100 µCi/mL [³H]-serine were added and the cells incubated for an additional 24 hr at 37°C. Plasma membrane fragments from 10⁸ CaCo-2 cells were isolated by mechanical disruption and centrifugation. Membranes were solubilized in a solution of 5µg/mL bovine serum albumin/50mM sodium acetate/50mM Tris-HCl (pH 7.0)/0.5% Triton X-100. The solubilized membrane fractions were then subjected to DEAE column chromatography, followed by Sepharose C12B affinity chromatography and cholesterol esterase affinity chromatography. Briefly, the solubilized membrane fraction was passed over a DEAE Sephadex C25 bed and eluted as a 0.65M NaCl fraction. The collected fractions were diluted to 0.15M NaCl and passed over a DEAE column and then washed with 0.4M NaCl plus 4M guanidine-HCl (pH 7.0). The eluted fractions were pooled and chromotographed onto a Sepharose C12B column and and washed with guanidine HCl buffer. [³⁵S]- and [³H]- containing fractions were pooled and lyophilized.

Analysis using SDS/PAGE detected a labeled band of 150 to 160 kD. Glycosamino-glycosidase treatment cleaves the protein from bound sugar residues, leaving a 45 to 50 kD proteinaceous core that is sensitive to protease V_{g} treatment. Treatment of this protein prior to electrophoresis with heparinase releases the [³⁵S] label from the protein, indicating that the native protein is bound to heparin *in vivo*. This result supports the identification of this protein as the pancreatic cholesterol esterase receptor, as the human pancreatic cholesterol esterase protein is known to contain a heparin binding site (*see*, Kyger *et al.*, *ibid.*). The identification of this protein as human PCE^{R} was confirmed by showing that the labeled fraction binds to a cholesterol esterase affinity column and can be eluted with 4.2M NaCl + 30mM sodium laurocholate.

### EXAMPLE 2

### Differential Binding Assays Useful In Developing PCE^{R}Binding Modulating Agents from Complex Mixtures of Small Molecules

Complex mixtures of small molecules are assayed for the presence of agents capable of inhibiting or modulating binding of PCE to PCE^{R}. A fungal broth or a peptide library is obtained using methods well-known in the art. This mixture is contacted with a microtitre plate on which either PCE^{R} or cells expressing PCE^{R} are bound. Such microtitre plates are then incubated with the small molecule mixture at 37°C for about 10 min to about 4h. Mixtures are removed from each microtitre plate and each plate washed with phosphate buffered saline (PBS). Detectably-labeled PCE, purified using biochemical means or expressed using recombinant genetic means, are added to the microtitre plates and incubated at 37°C for 12h. The PCE-containing mixture is then removed, the plates washed repetitively with physiological saline or phosphate-buffered saline (PBS), and the amount of detectable label bound determined. Small molecules which inhibit or modulate PCE-PCE^{R}-binding are then eluted using a 2M NaCl solution, dialyzed against 100mM Tris-HCl (pH 7.5) and concentrated using routine biochemical procedures. Additional fractionation and characterization is accomplished using high pressure liquid chromatography as desired.

### EXAMPLE 3

### Differential Expression of the PCE^{R}and Compounds Effecting Such Differential Expression

Complex mixtures of small molecules are assayed for the presence of agents capable of inhibiting or modulating PCE^{R} gene expression. A fungal broth or a peptide library is obtained using methods well-known in the art. This mixture is first contacted with cells expressing PCE^{R} for a time and at a concentration sufficient to inhibit or modulate PCE^{R} gene expression; for CaCo-2 cells, such incubations are performed for about 12h. Such mixtures are then removed from each plate of cells and each plate washed with phosphate buffered saline (PBS). Detectably-labeled PCE purified using biochemical means or expressed using recombinant genetic means are added to the microtitre plates and incubated at 37°C for 4h. The PCE-containing mixture is then removed, the plates washed repetitively with physiological saline or phosphate-buffered saline (PBS), and the amount of detectable bound label determined. This is compared to the amount of detectable label bound by cells grown in the absence of the small molecule mixture. Small molecules which inhibit or modulate PCE^{R} gene expression are then fractionated from the mixture using conventional biochemical methods, and the binding experiments described herein performed repetitively on fractions of increasing homegeneity.

It should be understood that the foregoing disclosure emphasizes certain specific embodiments of the invention and that all modifications or alternatives equivalent thereto are within the spirit and scope of the invention as set forth in the appended claims.

## Claims

1. A homogeneous composition of matter comprising a mammalian pancreatic cholesterol esterase receptor protein.

2. The mammalian pancreatic cholesterol esterase receptor protein of claim 1 that is human pancreatic cholesterol esterase receptor protein having a native molecular weight of from about 150 to about 160 kilodaltons.

3. A method of screening a compound as an inhibitor of pancreatic cholesterol esterase binding to a mammalian pancreatic cholesterol esterase receptor, the method comprising the following steps:
(a) incubating human CaCo-2 cells that express the human pancreatic cholesterol esterase receptor protein at the cell surface with pancreatic cholesterol esterase protein in the presence or absence of the inhibitor compound; and
(b) comparing the extent of pancreatic cholesterol esterase protein binding in the presence of the inhibitor to the extent of pancreatic cholesterol esterase protein binding in the absence of the inhibitor.

4. A method of screening a compound as an inhibitor of pancreatic cholesterol esterase receptor expression *in vivo*, the method comprising the following steps:
(a) incubating human CaCo-2 cells with the inhibitor compound for a time sufficient to inhibit expression of the pancreatic cholesterol esterase receptor;
(b) further incubating the human CaCo-2 cells of subpart (a) with pancreatic cholesterol esterase protein; and
(c) comparing the extent of pancreatic cholesterol esterase protein binding to the human CaCo-2 cells of subpart (a) to the extent of pancreatic cholesterol esterase protein binding to human CaCo-2 cells that have not been incubated with the inhibitor compound.

5. A method of screening a compound as an inhibitor of pancreatic cholesterol binding to a mammalian pancreatic cholesterol esterase receptor, the method comprising the following steps:
(a) incubating the human pancreatic cholesterol esterase receptor protein of claim 1 with pancreatic cholesterol esterase protein in the presence or absence of the inhibitor compound; and
(b) comparing the extent of pancreatic cholesterol esterase protein binding to the receptor protein in the presence of the inhibitor to the extent of pancreatic cholesterol esterase protein binding to the receptor protein in the absence of the inhibitor.

6. A method according to claim 5, wherein the human pancreatic cholesterol esterase receptor protein is bound to a solid support.

7. A method according to claim 6, wherein the solid support is a polyacrylamide or agarose bead.

8. A method according to claim 6, wherein the solid support comprises the inner surface of a microtitre plate.

9. A method according to any one of claims 3 to 8, wherein the pancreatic cholesterol esterase is detectably labeled with a radioactive label, an antigenic label, a hapten label or a fluorescent label.

10. An inhibitor of pancreatic cholesterol esterase receptor expression isolated by a method according to claim 4 or claim 5.

11. An antibody or fragment thereof that is immunologically reactive to a mammalian pancreatic cholesterol esterase receptor protein.

12. An antibody according to claim 11, wherein the antibody is a monoclonal antibody.

13. An antibody according to claim 11 that is a chimeric antibody.

14. An antibody according to any one of claims 11 to 13, wherein the mammalian pancreatic cholesterol esterase receptor protein is the human pancreatic cholesterol esterase receptor protein.

15. A cell line which produces an antibody or fragment thereof that is immunologically reactive to a mammalian pancreatic cholesterol esterase receptor protein.

16. A cell line according to claim 15, wherein the antibody is a monoclonal antibody.

17. A cell line according to claim 15, wherein the mammalian pancreatic cholesterol esterase receptor protein is the human pancreatic cholesterol esterase receptor protein.

18. A pharmaceutical composition comprising a therapeutically effective amount of an antibody or fragment thereof according to any one of claims 11 to 14, in a pharmaceutically acceptable carrier.

19. An epitope of a mammalian pancreatic cholesterol esterase receptor protein wherein the epitope is immunologically reactive to an antibody or fragment thereof according to any one of claims 11 to 14.
